# EUROPEAN PATENT APPLICATION

(11) **EP 3 599 002 A2**
(43) Date of publication of application: **29.01.2020**
(21) Application number: 19197105.0
(22) Date of filing: 05.08.2015
(51) Int. Cl.: A63B 71/00, A47G 29/087, A63B 71/06, A61H 1/02, A63B 21/005, A63B 21/00, A63B 21/16, A63B 23/12, A63B 23/14, A63B 23/16, A63B 24/00, A63B 22/00

(54) **UPPER LIMB REHABILITATION SYSTEM**

(30) Priority: 08.08.2014 EP 14180356
(62) Divisional of application: 15750330.1
(71) Applicant: Ecole Polytechnique Fédérale de Lausanne (EPFL) EPFL-TTO, 1015 Lausanne (CH); Mindmaze Holding S.A., 1006 Lausanne (CH)
(72) Inventor: Rognini, Giulio, CH-1007 Lausanne (CH); Tadi Ravi, Tej, CH-1007 Lausanne (CH)
(74) Representative: Harrison IP Limited

(57) **Abstract**

The invention relates to an upper limb rehabilitation system for rehabilitating an upper limb of a subject, that comprises
(a) a rehabilitation device, comprising a mobile platform, a fixed platform (12), a forearm supporting structure (22) and a movement altering device (30, 31, 34, 38, 45, 46), wherein said mobile platform is movable in regard to said fixed platform (12), and wherein, in use, the upper limb of the subject is in physical contact with said forearm supporting structure (22) and able to exert a force against said forearm supporting structure (22), and wherein said movement altering device (30, 31, 34, 38, 45, 46) is operable to alter a movement of said mobile platform in response to said force;
(b) a controller interface for controlling said movement altering device (30, 31, 34, 38, 45, 46);
(c) a programmable computer for controlling said controller interface, said programmable computer being operable to construct a VR environment and to provide haptic feedback to the subject through said controller interface; and
(d) a VR display for the subject to view the VR environment.

## Description

### FIELD OF THE INVENTION

The present invention relates to the field of rehabilitation systems, more particularly to an upper limb rehabilitation system for rehabilitating the upper limb of a subject. The present invention further relates to an integrated rehabilitation platform that combines robotics and interactive gaming to enable individual undergoing rehabilitation to improve performance of coordinated movements of the shoulder, the forearm and the hand.

### BACKGOUND OF THE INVENTION

In the last 15 years, technological development has mainly focused on two kinds of rehabilitation systems: robotic systems and sensor based systems. Sensor based systems target to develop small portable devices enabling to monitor functional movement in natural settings like at home. However, only subjects with a certain level of strength can fully profit from sensor based systems. Robots on the other hand have shown to be able to facilitate repetitive training in stroke subjects with all functional levels which has proven to address brain plasticity improving sensorimotor function. Moreover, robotic systems, such as the MIT-Manus, the Haptic Master and the MIME, guarantee a large variability of goal-tailored training exercises which encourages subjects to use all their capabilities to improve their motor performances.

A recent review paper on rehabilitation technology advocates for a general improvement in short-term and long-term strength of upper limbs movements after training with robotics. Inversely, experimental evidence speaks against an improvement of the activity level. This might be because most rehabilitation systems support analytical training methods rather than task-oriented training. Indeed, studies using neuroimaging techniques have shown that functional recovery from stroke is positively affected by task-specific arm/hand sensorimotor input characterizing training or everyday use. Moreover, motor control studies have suggested that movements are planned as the combination of a relatively small number of muscle co-contraction patterns called synergies. Currently, only few systems are able to provide task-oriented exercises for the upper extremity. Of these systems, only ADLER allows for training of the entire arm and hand (but without an actuated hand gripping tool) and despite the MIT-MANUS team recently developed a hand module to complete the previous systems, this will not allow to train all joints of the upper limb at the same time. Another robotic system as disclosed in US2008/0161733 allows three dimensional arm movements but not actuated and coordinated reaching-grasp movements. Therefore, these solutions are still not offering the training of movement strategies as needed during real life arm-hand performance.

US2006/0106326 discloses wrist and upper extremity motion system which includes a series of motors that can apply torques to a wrist about the tree axes of wrist rotation: pronation/supination, flexion/extension, and adduction/abduction. In particular, the pronation/supination (PS) axis extends parallel to the longitudinal axis of the device. Rotation of the device about the PS axis will cause or result from pronation and supination of the subject's wrist and arm. The flexion/extension (FE) axis extends through the subject's wrist perpendicular to the PS axis. Rotation of the system about the FE axis will cause or result from flexion and extension of the subject's wrist. The abduction/adduction (AA) axis is perpendicular to the FE and PS axes and extends below the handle of the system. Rotation of the system about the AA axis will cause or result from abduction and adduction of the subject's wrist.

The configuration of this system is however mainly configured for the wrist rehabilitation of a subject. This system is therefore not adapted to simulate for example an interaction with a virtual object which is translating and rotating about an axis passing by its centre of gravity for training the hand and fingers' movements of the subject, thereby reducing the capability of the system to help retraining the whole functions of the impaired upper limb.

Accordingly, an aim of the present invention is to provide an upper limb rehabilitation system to address problems such as these in the prior art.

### SUMMARY OF THE INVENTION

According to a first aspect of the invention, there is provided an upper limb rehabilitation system as hereinafter set forth in Claim 1 of the appended claims.

Preferred features of the first aspect of the invention are set forth in Claims 2 to 11 of the appended claims.

According to a second aspect of the invention, there is provided an upper limb rehabilitation system as set forth in Claim 15 of the appended claims.

### BRIEF DESCRIPTION OF FIGURES

The invention will be better understood thanks to the following detailed description of the invention with reference to the attached figures, in which:
- FIG. 1 shows a perspective view of the upper limb rehabilitation system according to the invention in a neutral/resting position,
- FIG. 2 shows a perspective view of the handle assembly of the upper limb rehabilitation system in a neutral/resting configuration,
- FIG. 3 shows another perspective view of the handle assembly in which the gripping device is in a first active position,
- FIG. 4 shows a further perspective view of the handle assembly in which the gripping device is in a second active position,
- FIG. 5a shows a perspective view of the gripping device of the handle assembly in an deployed/resting position,
- FIG. 5b shows a perspective view of the gripping device in a retracted position,
- FIG. 5c shows a front view of a foldable structure of the gripping device,
- FIG. 5d shows a detailed perspective view of one of two complementary parts of the gripping device,
- FIG. 6 shows a top view of the upper limb rehabilitation system of FIG. 1,
- FIG. 7 shows a top view of the upper limb rehabilitation system in a first active position,
- FIG. 8 shows a top view of the upper limb rehabilitation system in a second active position,
- FIG. 9 shows a top view of the upper limb rehabilitation system in a third active position,
- FIG. 10 shows a top view of the upper limb rehabilitation system in a forth active position,
- FIG. 11 shows the upper limb rehabilitation system clamped on a table,
- FIG. 12 shows a perspective view of the fixed platform of the upper limb rehabilitation system comprising gravity compensation means,
- FIG. 13 shows the subject's hand operating the handle assembly of the rehabilitation system,
- FIG. 14a and FIG. 14b show a detailed view of a motor transmission arranged on a first, respectively a second degree of freedom of the rehabilitation system,
- FIG. 15a and FIG. 15b show a braking device in a unactuated and actuated configuration respectively.

### DETAILED DESCRIPTION OF THE INVENTION

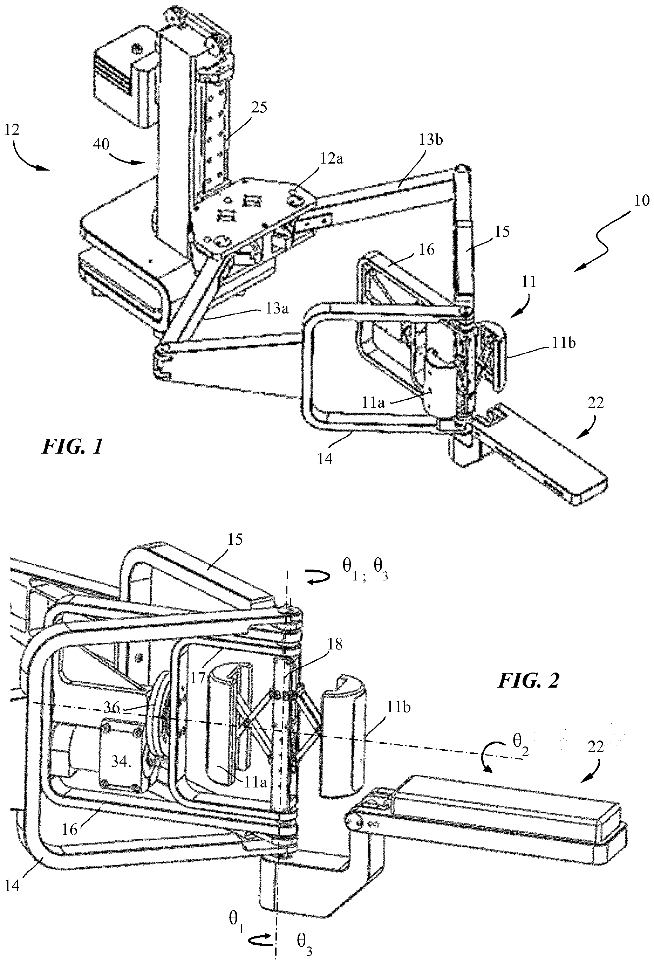

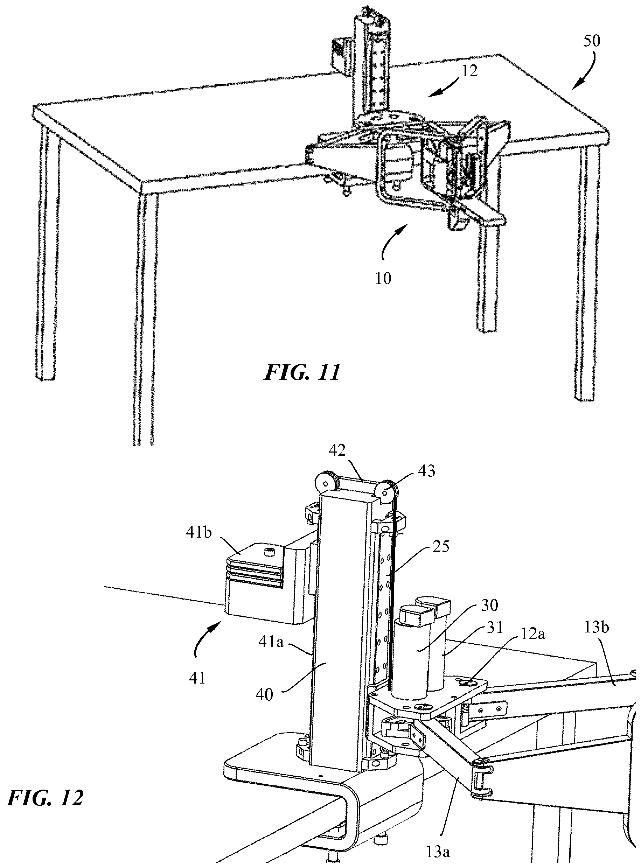

An upper limb rehabilitation system, for improving performance of coordinated movements of an upper limb (i.e. shoulder, elbow, wrist and hand) of a subject, is described herein and is seen generally in Figure 1. The compact and lightweight characteristics of the system allow it to be used in any restricted-space area in medical and rehabilitative facilities. In addition, the upper limb rehabilitation system is advantageously transportable and configured to be easily clamped to any appropriate supporting structure such as a table 50 as shown in Figure 11 allowing on-site therapeutic procedures at home.

As particularly shown in Figure 1, the rehabilitation system comprises essentially a gripping device 11 mounted on an articulated handle assembly 10 which is pivotally connected to a fixed platform 12 in a manner that the gripping device 11 is movable within a plane which is substantially coplanar or parallel to the transverse plane of the subject, referred hereafter as the working plane, during a working session. The handle assembly 10 is supported by a holding unit 12a which is slidably mounted along a linear guide 25 arranged vertically on a tower 40 forming part of the fixed platform 12. The holding unit 12a comprises locking means (not shown) in order to be locked in an appropriate height in function of the supporting structure and/or the position of the subject to adjust the working plane at a desired height. In a variant, the holding unit 12a of the rehabilitation system is arranged to freely move upwards and downwards along the linear guide 25 to provide a further degree of freedom to the system thereby allowing the subject to perform exercises in a three dimension environment with partial or full compensation of his forearm thanks to gravity compensation means as described subsequently.

The handle assembly 10 comprises a first and a second holding element 14, 15 which have each a general ⊏- shaped construction and which are each oriented within a plane perpendicular to the plane of the supporting structure 50 on which the fixed platform 12 of the rehabilitation system is clamped. These first and second holding element 14, 15 are pivotally connected to a supporting structure which comprises a first and a second connecting link 13a, 13b which are both pivotally connected to the holding unit 12a of the fixed platform 12 to allow movements of the handle assembly 10 in two orthogonal directions within the working plane.

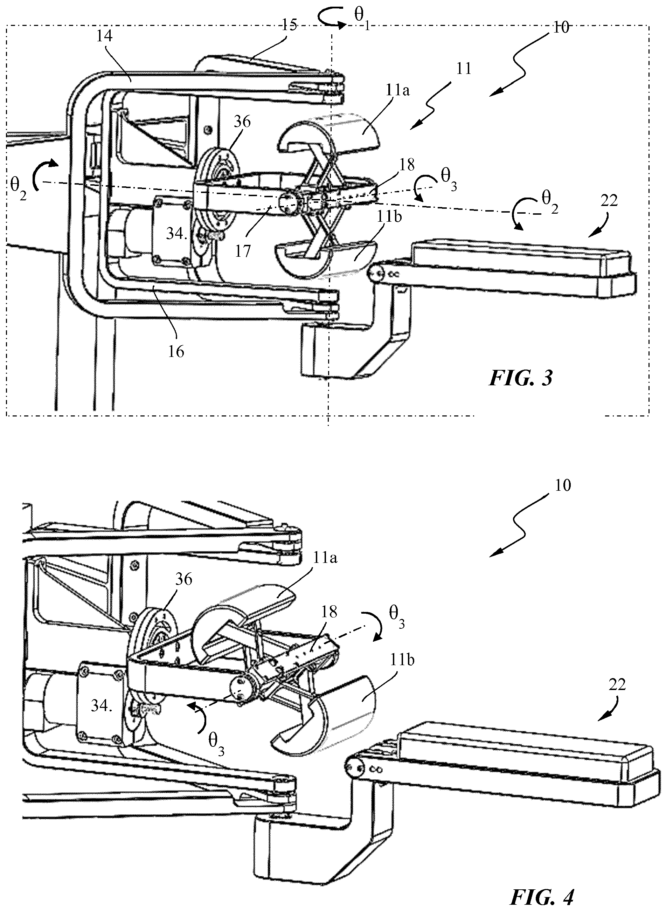

Referring to Figure 2, the extremities of the upper and lower parts of the first holding element 14 are pivotally connected to the corresponding extremities of respective upper and lower parts of the second holding element 15 such that said first and second holding elements 14, 15 of the handle assembly 10 are both rotatable about a first axis of rotation θ₁ which is perpendicular to the transverse plane of the subject. Referring now to Figure 3, the handle assembly 10 further comprises a third and a fourth holding element 16, 17, both of which have also a general ⊏- shaped construction. The extremities of lower and upper parts of the third holding element 16 are pivotally connected to respective upper and lower parts of the first and second holding elements 14, 15. The gripping device 11 is mounted on a shaft 18 whose extremities are rotatably mounted on opposite sides of the fourth ⊏- shaped holding element 17. The latter is pivotally connected to the third holding element 16 to rotate about a second axis of rotation θ₂ perpendicular to the first axis of rotation θ₁ within said third holding element 16 in order to allow forearm pronation movements and forearm supination movements of the subject through an angle up to +/- 90 degrees as shown in Figure 3 as well as wrist flexion movements about a third axis of rotation θ₃ as shown in Figure 4 corresponding to the longitudinal/central axis of the shaft 18. According to the configuration of the system, the first and second axes of rotation θ₁, θ₂ as well as the longitudinal/central axis of the shaft 18 of the gripping device 11 always intersect at one point independently from the orientation of each of the first, second, third and fourth holding elements 14, 15, 16, 17.

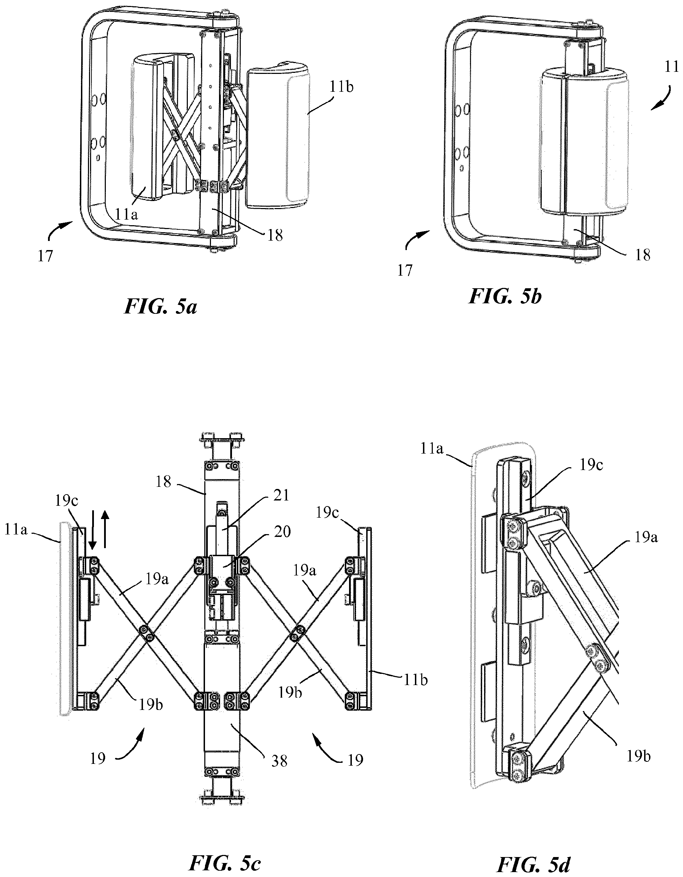

Turning now in particular to Figures 5a to 5d, the gripping device 11 of the handle assembly 10 comprises two complementary parts 11a, 11b which are each connected to a foldable structure 19 (Figure 5c) mounted on both sides of the shaft 18. Each foldable structure 19 comprises a first and a second intersecting link 19a, 19b pivotally mounted together at their centre. The first intersecting link 19a is further connected at one end to the shaft 18 while its other end is slidably mounted along a rail 19c arranged on the upper inner side of the corresponding complementary part 11a, 11b of the gripping device 11. The second intersecting link 19b, for its part, is connected at one end to the lower inner side of said complementary part 11a, 11b while its other end is connected to a travel nut 20 of a ball screw 21 mounted inside the shaft 18. Each foldable structure 19 is folded between the shaft 18 and the inner side of the corresponding complementary part 11a, 11b of the gripping device 11 when the latter is in a retracted position as shown in Figure 5b. The configuration of the gripping device allows hand grasp movements and hand release movements of the subject.

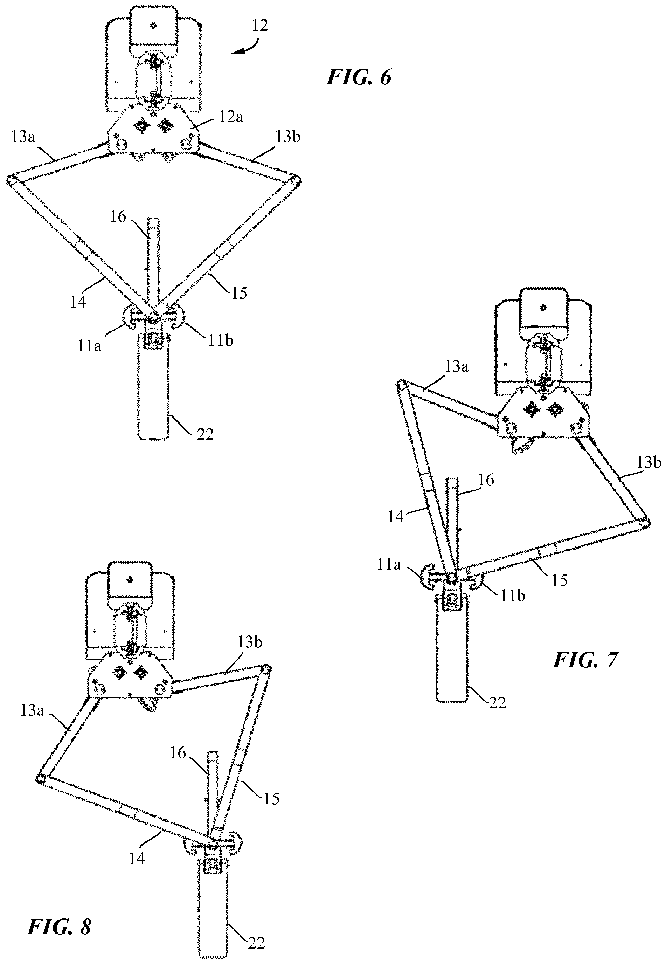

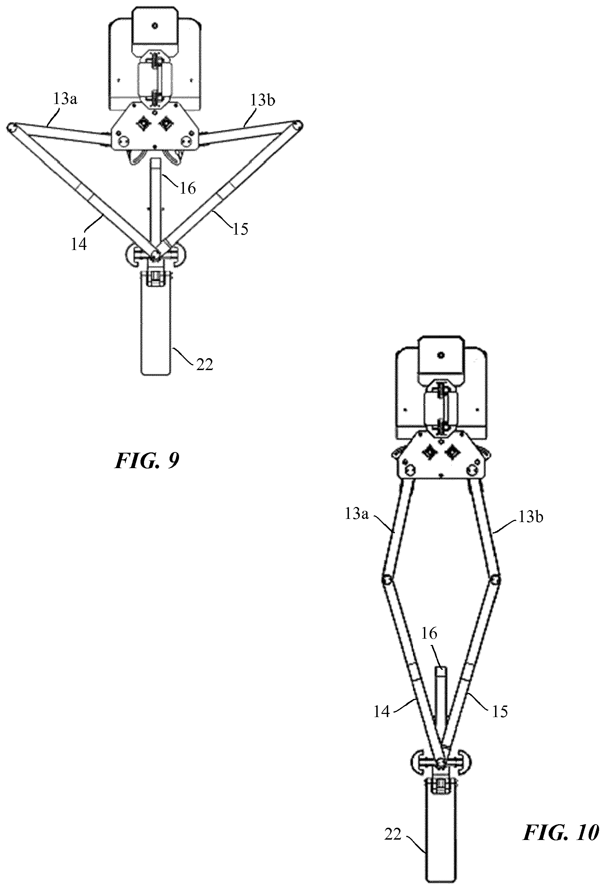

The upper limb rehabilitation device according to the invention further comprises a forearm supporting structure 22 which is pivotally connected to handle assembly 10 to rest the subject's forearm, during a rehabilitation session, when operating the handle assembly 10 in the two orthogonal directions within the working plane as shown specifically in Figures 6 to 10.

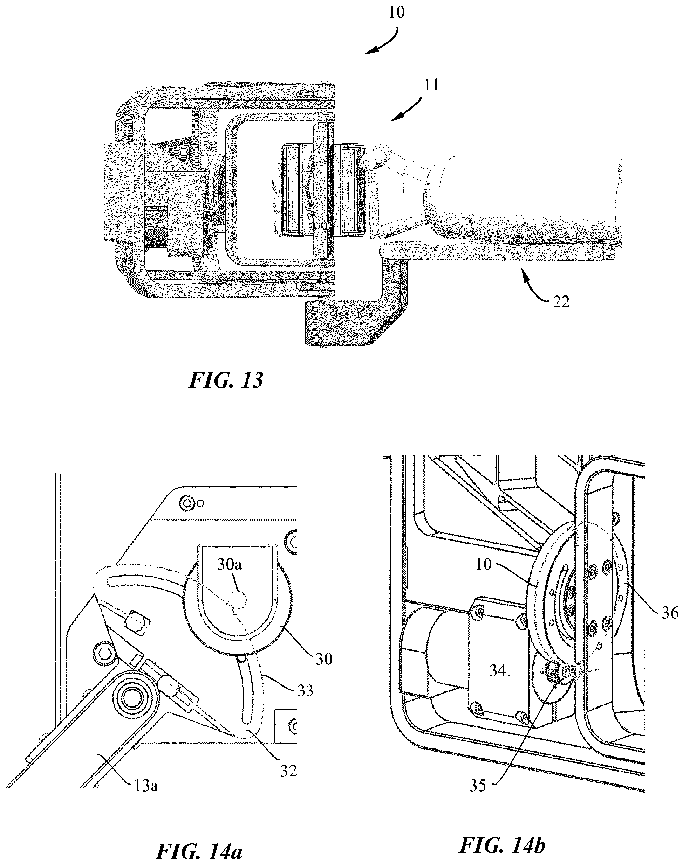

According to one embodiment of the invention, the upper limb rehabilitation system as described above is adapted to provide active forces and/or torques to assist the subject's motions as well as resistive active movement in response to the subject motion to simulate the interaction with a virtual object. More specifically, the rehabilitation system according to this embodiment comprises two motors 30, 31 as shown in Figure 12 coupled with the first, respectively the second connecting link 13a, 13b to assist or impede movements of the handle assembly 10 in the two orthogonal directions within the working plane. Figures 14a shows a detailed view of the motor 30 coupled to a driving pulley 30a which is connected to a driven pulley 32 through a cable/belt 33 and which is arranged at one extremity of the connecting link 13a. A third motor 34 is mounted on the third ⊏- shaped holding element 16 as shown for example in Figure 14b. A driving pulley 35 is mounted on the motor's shaft and is coupled to a driven pulley 36 through a cable/belt 37 to assist or impede rotational movement of the fourth holding element 17 along with the gripping device 11 about the second axis of rotation θ₂. The cable transmission configuration amplified the torque produced by the motors 30, 31 and 34 by a factor equal to the ratio between the radiuses of the driven and the drive pulley respectively. Cable transmissions are preferred over more standard solutions such as gear boxes which have the disadvantage of introducing friction and backlash preventing a smooth interaction of the rehabilitation system with the subject.

With reference to Figure 5c, a fourth motor 38 is arranged inside the shaft 18 of the gripping device 11 and is coupled to the ball screw 21 to assist or impede grasp movements of the subject's hand. More particularly, the motor 38 is configured to be driven to rotate the ball screw 21 clockwise or anticlockwise to cause the travel nut 20 to move upwards or downwards thereby bringing the two complementary parts 11a, 11b of the gripping device, through the foldable structure 19, against each other or moving said complementary parts 11a, 11b away from each other.

According to this embodiment, the rehabilitation system can advantageously be used in an integrated robotic platform that combines robotics and interactive gaming to facilitate performance of task-specific repetitive, upper extremity/hand motor tasks, to enable individual undergoing rehabilitation to improve performance of coordinated movements of the forearm and hand. To this end, the robotic integrated platform comprises a gaming interface which is provided with a display device to simulate virtual reality and to present sensorimotor integration tasks to the subject. The rehabilitation device is used as a haptic interface to simulate the interaction with objects in a virtual reality world presented in the display device. Positions sensors (not shown) such as encoders or potentiometers are placed on the rehabilitation system on strategic locations to track the movement of the system. The integrated robotic platform further comprises a controller interface that is adapted to monitor and forward continuously, during the rehabilitation session, the outputs from the sensors to a programmable computer which determines desired force feedback to be applied by the controller interface to the corresponding motors 30, 31, 34 and 38 of rehabilitation system.

The level of assistance can be tuned on-line (e.g. during a working session) providing adapting time-dependent force fields. For example, given a specific task in which the subject has to follow a certain trajectory, the robotic platform can provide different level of support, from complete assistance, i.e. movements of the rehabilitation system are entirely driven by its motors, to full transparency, i.e. movements of the rehabilitation system are entirely caused by the subject upper limb's movements. The robotic platform can also provide force perturbation to increase the difficulty of the task.

The robotic integrated platform optionally comprises a controller arranged to monitor different physiological signals of the subject such as EEG or EMG and to drive the motors of the rehabilitation system for real time correlation between the movement of the device and brain activity pattern of the subject.

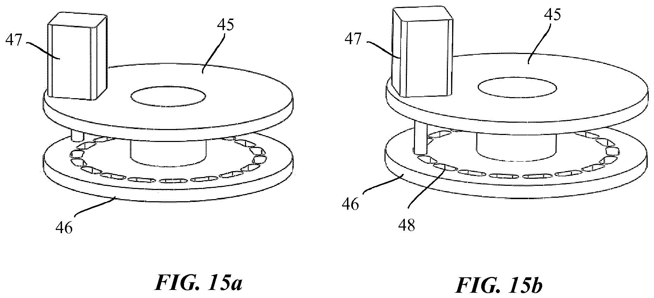

According to one alternate embodiment of the invention, the upper limb rehabilitation system as described above is adapted to provide resistive movements only, in response to the subject's motions. In this configuration, the motors are replaced by braking devices. For example, forearm pronation movements and forearm supination movements of the subject around the second axis of rotation θ₂ can be hindered by a braking device as shown in Figures 15a, 15b. The braking device comprises for example a fixed part 45 mounted on the third holding element of the rehabilitation system (not shown) and a rotating part 46 rotatably mounted on the fixed part 45 and fixed to the fourth holding element in order to rotate about the second axis of rotation θ₂ of the system. A linear actuator 47 is mounted on the fixed part 45 to engage upon actuation into one of several arcuate slots 48 disposed along a circular path on the rotating part 45 thereby stopping its rotation in order to simulate a physical contact with a virtual object.

The rehabilitation system according to the invention preferably comprises gravity compensation means that can provide different levels of gravity compensation either passively (i.e. counterweights or springs) or actively (i.e. motors). Figure 12 illustrates an example of gravity compensation means which comprise a counterweight holder 41 slidably mounted along a vertical rail 41a on the rear side of the tower 40 of the fixed platform 12. At least one cable 42 is arranged on pulleys 43 located on the top of the tower 40 to connect the holding unit 12a with the counterweight holder 41. The latter is adapted to receive one or several counterweight 41b in function of the desired level of gravity compensation.

A related method for the rehabilitation of an upper limb of a subject comprises the steps of:
i) mapping the movements of the subjects into virtual or augmented reality environments in which the subject receive visual feedback concerning his/her own movements, for example through the projection of an avatar replicating his/her movements, as well as visual feedback about a virtual/augmented environment that includes several settings and objects whose physical properties (e.g. stiffness) are simulated through the upper limb rehabilitation system or through any other haptic display (able to render temperature, roughness etc) coupled with the system or in contact with the subject;
ii) recording physiological signals of the subject such as electroencephalography (EEG), functional magnetic resonance imaging (fMRI), functional near-infrared spectroscopic imaging (fNRIS), electrocardiography (ECG), skin conductance or electromyography (EMG) as well as movements parameters such as trajectories, velocities, accelerations and forces when the subject is operating the upper limb rehabilitation system; and
iii) controlling the movements of said system in function of the recorded physiological, brain or movement signals.

Another method for the rehabilitation of an upper limb of a subject comprises the steps of (i) recording physiological signals of the subject such as electroencephalography (EEG), functional magnetic resonance imaging (fMRI), functional near-infrared spectroscopic imaging (fNRIS) or Electromyography (EMG) when the subject is operating the upper limb rehabilitation system, and (ii) interfering with the movements of said system in function of the recorded physiological signals.

## Claims

1. An upper limb rehabilitation system for rehabilitating an upper limb of a subject, comprising:
(a) a rehabilitation device, comprising a mobile platform, a fixed platform (12), a forearm supporting structure (22) and a movement altering device (30, 31, 34, 38, 45, 46), wherein said mobile platform is movable in regard to said fixed platform (12), and wherein, in use, the upper limb of the subject is in physical contact with said forearm supporting structure (22) and able to exert a force against said forearm supporting structure (22), and wherein said movement altering device (30, 31, 34, 38, 45, 46) is operable to alter a movement of said mobile platform in response to said force;
(b) a controller interface for controlling said movement altering device (30, 31, 34, 38, 45, 46);
(c) a programmable computer for controlling said controller interface, said programmable computer being operable to construct a VR environment and to provide haptic feedback to the subject through said controller interface; and
(d) a VR display for the subject to view the VR environment.

2. An upper limb rehabilitation system according to Claim 1, wherein said movement altering device (30, 31, 34, 38, 45, 46) further comprises at least one motor (30, 31, 34, 38) operable to exert a force on said mobile platform and wherein preferably the system further comprises at least one position sensor for tracking the movement of the mobile platform, and the programmable computer is operable to determine a desired force feedback to be applied by the controller interface to the or each motor (30, 31, 34, 38).

3. An upper limb rehabilitation system according to Claim 1 or 2, wherein said movement altering device (30, 31, 34, 38, 45, 46) further comprises at least one brake (45, 46) operable to exert a force on said mobile platform.

4. An upper limb rehabilitation system according to any preceding claim, further comprising at least one physiological sensor for detecting a physiological signal of the subject, and wherein the programmable computer is operable to drive the or each motor of the rehabilitation system in dependence on the physiological signal for real time correlation between the movement of the device and brain activity pattern of the subject.

5. An upper limb rehabilitation system according to Claim 2, further comprising at least one physiological sensor for detecting a physiological signal of the subject, and a physiological monitor for receiving said physiological signal and for transmitting said physiological signal to said programmable computer, wherein said VR module is operable to adjust said VR environment according to said physiological signal, and wherein preferably said physiological sensor comprises one or more of an electroencephalogram (EEG) sensor, electromyogram (EMG) sensor, electrooculography (EOG) sensor, electrocardiogram (ECG) sensor, functional magnetic resonance imaging (fMRI) sensor, functional near-infrared spectroscopic imaging (fNRIS) sensor, and skin conductance sensor.

6. An upper limb rehabilitation system according to any preceding claim, wherein said mobile platform comprises a gripping device for gripping by the subject.

7. An upper limb rehabilitation system according to any preceding claim, wherein said mobile platform comprises an articulate handle assembly movable, during a rehabilitation session, within a plane which is substantially coplanar or parallel to the transverse plane of the subject, wherein the handle assembly is provided with a gripping device comprising a shaft, wherein said articulate handle assembly comprises:
(a) a supporting structure having a first and a second holding elements pivotally connected together to rotate about a first axis of rotation (θ1) perpendicular to said transverse plane,
(b) a third holding element connected to said supporting structure, and
(c) a fourth holding element supporting the shaft of the gripping device and pivotally connected to said third holding element to rotate about a second axis of rotation (θ2) which is perpendicular to the first axis of rotation (θ1),
and in that said first and second axis of rotation (θ1, θ2) as well as the central axis (θ3) of the shaft of the gripping device always intersect at one point independently from the orientation of the first, second, third and fourth holding elements.

8. An upper limb rehabilitation system according to Claim 7, wherein each of said first and second holding elements (14, 15) of the supporting structure comprises upper and lower parts which are parallel to said transverse plane of the subject, wherein the upper part and lower parts of the first holding element (14) are pivotally connected respectively to the upper and lower parts of the second holding element (15) such that said first and second holding elements (14, 15) are both rotatable about said first axis of rotation (θ1), and wherein preferably said third holding element (16) comprises upper and lower parts which are parallel to said transverse plane of the patient and pivotally connected to respective upper and lower parts of the first and/or second holding elements (14, 15)

9. An upper limb rehabilitation system according to Claim 7 or 8, wherein said first, second, third and fourth holding element (14, 15, 16, 17) have a general C-shaped or ⊏-shaped construction.

10. An upper limb rehabilitation system according to any one of Claims 7 to 9, wherein the gripping device (11) of the handle assembly (10) comprises two ergonomic parts (11a, 11b) and two foldable structure (19) therebetween, the two ergonomic part (11a, 11b) being spaced apart in a resting position and arranged to be squeezed against each other by the hand grasp movements of the subject, wherein each foldable structures (19) is connected to the shaft (18) of the gripping device (11) and to a travel nut (20) of a ball screw (21) mounted inside said shaft (18), each foldable structure being further slidably mounted on the inner side of the corresponding complementary part (11a, 11b) of said gripping device (11).

11. An upper limb rehabilitation system according to Claim 10, wherein each foldable structure (19) comprises two interconnected links (19a, 19b) pivotally mounted together at their centre, wherein one extremity of one intersecting link (19a) of each foldable structure (19) is connected to the shaft (18) of the gripping device (11), the other extremity of said one intersecting link (19a) being slidably mounted along a rail (19c) arranged on the inner side of the corresponding complementary part (11a, 11b) of the gripping device (11), and wherein one extremity of the other intersecting link (19b) is connected to the inner side of said complementary part (11a, 11b), the other extremity of said other intersecting link (19b) being connected to a travel nut (20) of a ball screw (21) mounted inside the shaft (18) of the gripping device (11).

12. An upper limb rehabilitation system according to any one of Claims 7 to 11, wherein a motor (34) is mounted on the third holding element (16) to assist and/or impede the rotation of the fourth holding element (17) about said second axis of rotation (θ2) and wherein preferably said system comprises a motor (38) arranged inside the shaft (18) of the gripping device (11), and said motor (38) is coupled to the ball screw (21) to assist or impede grasp movements of the subject's upper limb.

13. An upper limb rehabilitation system according to any one of Claims 7 to 13, wherein said system comprises a braking device, wherein said braking device comprises a fixed part (45) mounted on the third holding element (16) and a rotating part (46) rotatably mounted on the fixed part (45) and connected to the fourth holding element (17) in order to rotate about said second axis of rotation (θ2), the braking device further comprising an actuator (47) configured to come into contact, upon actuation, with the rotating part (45) thereby stopping its rotation in order to simulate a physical contact with a virtual object.

14. An upper limb rehabilitation system according to any preceding claim, further comprising a controller for monitoring EEG or EMG signals of the subject, and for controlling said movement altering device to provide force feedback according to said signals.

15. An upper limb rehabilitation system for rehabilitating an upper limb of a subject, comprising:
i) means for mapping the movements of the subjects into virtual or augmented reality environments in which the subject receives visual feedback concerning his/her own movements as well as visual feedback about a virtual/augmented environment whose physical properties are simulated through a haptic feedback system in contact with the subject;
ii) means for recording physiological signals such as brain signals of the subject as well as movement parameters of the haptic feedback system when the subject is operating said system; and
iii) means for controlling the movements of said system in function of the recorded physiological signals or movement parameters.
